# NEW EUROPEAN PATENT SPECIFICATION

(11) **EP 2 590 876 B2**
(45) Date of publication and mention of the opposition decision: **22.08.2018**
(45) Mention of the grant of the patent: 03.12.2014
(21) Application number: 10768260.1
(22) Date of filing: 25.10.2010
(51) Int. Cl.: B65D 85/804

(54) **A capsule for preparation of a food product from a food preparation machine**
Kapsel zur Herstellung eines Lebensmittelproduktes aus einer Lebensmittelherstellungsmaschine
Capsule de préparation de produits alimentaires pour machine de préparation d'aliments

(30) Priority: 07.07.2010 EP 10168664
(43) Date of publication of application: 15.05.2013
(73) Proprietor: Nestec S.A., 1800 Vevey (CH)
(72) Inventor: DOLEAC, Frédéric, 25160 Vaux et Chantegrue (FR); RAEDERER, Marc, Dublin, OH 43017 (US)
(74) Representative: Rupp, Christian
(86) International application number: PCT/EP2010/066067
(87) International publication number: WO 2012/003891

(56) References cited:
- WO-A1-2005//020769
- WO-A1-2006//037062
- WO-A1-2009//106598
- WO-A2-2006//021405
- DE-A1-102004 056 224
- DE-A1-102004 056 317

## Description

### Field of the invention

The present invention concerns a capsule for use with a food preparation machine by extraction and/or dissolution of a food ingredient contained therein.

### Background of the invention

Machines for preparing a beverage or other types of food product by injecting pressurized fluid into a capsule are well-known, especially in the field of producing coffee or coffee type beverages. In addition, other substances such as chocolate or milk products can be extracted or dissolved to form a beverage, as well as other types of nutritional products such as infant nutrition products. The advantages of such a system are in particular the conservation and freshness of the ingredients, as well as the possibility of facilitating the operations of preparing the beverage.

The method of preparing the beverages using such a machine is in principle as follows. The capsule is usually first inserted into a receiving chamber of the beverage preparation machine. After that, a water injecting means, such as a needle connected to the liquid supply of the machine and which projects inside the receiving chamber, is introduced through a wall, typically a top wall, of the capsule to inject a hot or cold liquid, in order to make the liquid interact with the ingredients within the capsule. The food product, typically a liquid beverage, resulting from this interaction is then discharged through a delivery, typically a bottom wall of the capsule. For example, the delivery wall opens due to the internal pressure in the capsule generated by injecting the liquid.

DE102004056317 is a German patent application to René Schifferle. It discloses a beverage machine for the preparation of a hot beverage by extraction of an ingredient contained in a capsule. The machine comprises an extraction chamber and a closure member for closing the chamber. The lower cup-shaped side of the extraction chamber serves as a positive receptacle for the capsule. Each of the lower side of the extraction chamber and the closure member comprises an injection element which is arranged for reciprocal perforation of the capsule.

When the extraction chamber is closed and locked, both ends of the capsule are perforated by an injection element. The capsule comprises top and bottom inner filtering means spaced apart respectively from the capsule top and bottom walls being pierced by the injection elements. The inner filtering means allow the circulation of extraction fluid through the ingredient, without said ingredient being moved throughout the capsule.

The extraction fluid is usually injected inside the capsule under pressure. In some instances, when the consumer removes the capsule, the fluid remaining inside the capsule can be forced back outside through the capsule top wall, which is called "backflow". Such a phenomenon is highly undesirable since the consumer may be hurt if spilled with hot water from the capsule. It was found that such a phenomenon is particularly frequent when the capsule comprises compartmented spaces therein.

DE102004056224 is a German patent application to Tchibo GmbH. It discloses a coffee machine for brewing a hot drink, in particular, a coffee drink, by means of a portion capsule with a capsule lid and a capsule base, comprising a pressurised water device, a portion capsule holder and a controller. The portion capsule holder is provided with two relatively-displaceable puncture means, which pierce the capsule lid and the capsule base on or after insertion of the portion capsule in the portion capsule holder, such that hot water can enter the portion capsule through the capsule lid and the drink escapes through the capsule base from the portion capsule. The controller is provided for the provision of three differing pressures for the pressurised water, for example, for brewing espresso, white coffee and filter coffee. The capsule base of the portion capsule can comprise a pre-weakened spot.

It should be understood that the interaction between the liquid and the ingredients within the capsule can be e.g. dissolving, extraction, brewing or any other interaction in order to prepare a beverage by means of ingredients provided within the capsule.

Furthermore, once the injection needle has pierced a wall of the capsule and injection of fluid has begun, the distal end of the needle which protrudes inside the capsule chamber is in contact with the product being prepared therein. After the product preparation is completed and needle has been removed from the capsule, food product can remain on the surface of the said needle, and cross contaminate a product preparation which would occur afterwards. In some cases when the machine is not used for a long time, bacterial growth may occur in the distal portion of the needle which was in contact with product, which is of course highly undesirable to the consumer.

It is therefore a main objective of the present invention to provide a capsule for preparing a food product using a food preparation machine that is convenient to use for consumer, that provides good dissolution and/or extraction properties of the ingredients contained therein, and which avoids backflow and contamination of the fluid injection needle of the machine.

### Summary of the invention

The present invention meets the objective cited above with a capsule as claimed in claim 1.

The fact that the showering means is built-in with the capsule top wall makes the capsule particularly easy and cheap to manufacture: there is no need for additional parts to be manufactured separately and then assembled with the top membrane, or inside the capsule.

The showering means comprises a plurality of showering holes having each a diameter which is inferior to the output diameter of the needle.

In any case, the diameter of the showering hole(s) is preferably less than the diameter of the needle outlet opening, and the speed of the jet of fluid through the top wall inside the chamber has a reduced speed compared to the speed of the jet coming out of the needle.

In a first embodiment of the present invention, the top wall is rigid and the showering means comprise a built-in portion protruding downwards inside the capsule chamber.

Preferably in that case, the top wall is pivotally attached to the capsule side walls via a pivot hinge.

In a second possible embodiment of the present invention, the top wall is a flexible film sealed onto the circumferential edges of the capsule body, said top wall comprising at least one layer of a pre-pierced film that is flexible enough to deform without piercing when in contact with the injection needle.

### Brief description of the drawings

Additional features and advantages of the present invention are described in, and will be apparent from, the description of the presently preferred embodiments which are set out below with reference to the drawings in which:
**Figure 1** is a schematic profile cut view of a capsule, used with a cup-shaped capsule holder;
**Figure 2** is a schematic view similar to figure 1, wherein the capsule is used with a ring shaped capsule holder;
**Figure 3** is a schematic profile cut view of an alternative construction of the capsule illustrated in figures 1 and 2;
**Figure 4** is a schematic profile cut view of a capsule, used with a cup-shaped capsule holder;
**Figure 5** is a schematic view similar to figure 3, wherein the capsule is used with a ring shaped capsule holder;
**Figure 6** is a schematic perspective view of a capsule with peelable closing membranes;
**Figure 7** is yet a schematic cut view of an embodiment of the invention.

### Detailed description of the invention

In the following detailed description, the example given is that of a machine - not represented in the drawing - with capsules for preparing beverages, typically coffee. Said machine comprises a fluid injection needle supported by an injection plate, for injection of a single jet of fluid inside the capsule, said needle being adapted in shape and size to protrude inside the capsule when said capsule holder is inserted into the machine in order for operation. Such a machine was described for instance in prior European patent EP 1967069 B1. According to the invention, the outer surface of the capsule top wall is in contact, at least partially, with the needle plate of the machine, during the dissolution/extraction step.

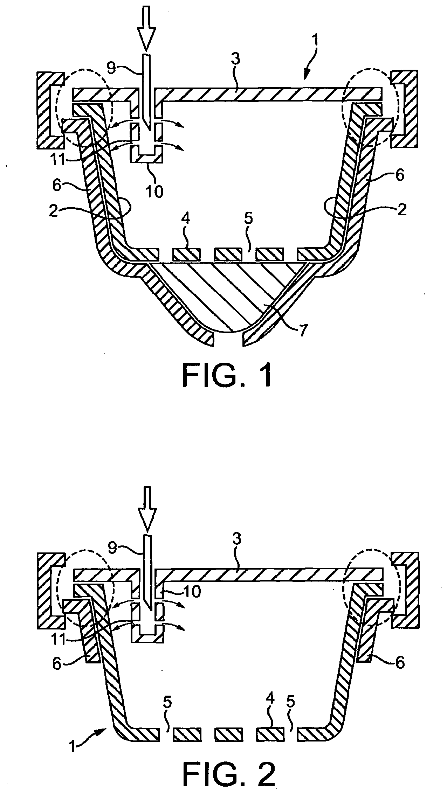

**Figure 1** shows a capsule 1, wherein the capsule body is cup-shaped, with side walls 2, a top wall 3, and a bottom wall 4 that is pierced with a plurality of dispensing holes 5 for the product produced inside the capsule chamber to flow out. The capsule top wall 3 is rigid, the outer surface of the rigid top wall is covered during storage by a removable lid, which can be a glued or sealed foil, or a semi-rigid lid clipped thereonto - not shown on the drawing -.

The capsule 1 is placed in a capsule holder 6, to which a supporting insert 7 can be added in order to support the bottom wall 4 of the capsule. Such an insert 7 can be necessary to prevent damage to the capsule wall, particularly when the capsule contains such products like roast and ground powdered coffee, which requires high fluid pressure inside the capsule chamber to prepare the final product, for instance espresso coffee. In this case, if the capsule that is loaded into the capsule holder does not fit properly the internal shape and size of the capsule holder, when fluid pressure inside the capsule holder increases, the portion of the capsule wall that is not supported by the capsule holder walls can be pushed outwardly, and even can be pierced, which is of course highly undesirable.

The insert 7 also has another important effect: in the case shown in figure 1, the said insert 7 counterbalances the flow of product that flushes out of the capsule dispensing holes 5. By doing this, the insert 7 contributes to keeping a high pressure inside the capsule chamber, while allowing the product by high pressure extraction to flow out.

Such an insert is not necessary in case the ingredient contained inside the capsule is meant to be dissolved or brewed at a low pressure, for instance in the case of soluble milk, tea, soup, soluble coffee, or roast and ground powdered coffee beans to be brewed at low pressure to produce "filter coffee"-like product.

As illustrated in **figure 1**, the capsule 1 comprises a top circumferential edge 8 disposed at the upper portion of the side walls 2, such that when said capsule is loaded in the capsule holder 6 and said capsule holder is placed in the machine in order for operation, the interface between the needle plate, the capsule holder 6, and the capsule edge 8 is leaktight so that fluid injected through the needle 9 can flow only through said capsule. More precisely, the top wall 3 is sealed onto said circumferential edges 8.

As illustrated in **figure 1****,** the top wall 3 comprises built-in showering means. The showering means 10 is a portion protruding downwards inside the capsule chamber that is built-in with the rigid top wall 3, which comprises a plurality of showering holes having each a diameter which is inferior to the output diameter of the fluid injection needle 9. The showering means 10 is able to accommodate the needle 9 without being pierced by said needle, and it transforms the single jet of fluid from said needle 9 into a plurality of jets - indicated with arrows in the drawing - directed towards the inside of the capsule chamber.

The top wall 3 can be made of any suitable material such as coated cardboard, metal, plastic, rigid laminated film, or a combination thereof, but preferably, it is made by injection of a thermoplastic such as polyethylene or polypropylene.

**Figure 2** shows a variant of the capsule described above in reference to figure 1. In this case, the capsule 1 is supported by a ring-shaped capsule holder 6. As described above, the interface between the needle plate, the capsule holder 6, and the capsule edge 8 is leaktight so that fluid injected through the needle 9 can flow only through said capsule.

In this case, it can be seen that no insert supports the bottom wall 4 of the capsule 1, nor does an insert support the side walls 2. Along with the explanations given above, the capsule shown in **figure 2** is therefore meant for a low pressure preparation of a food product.

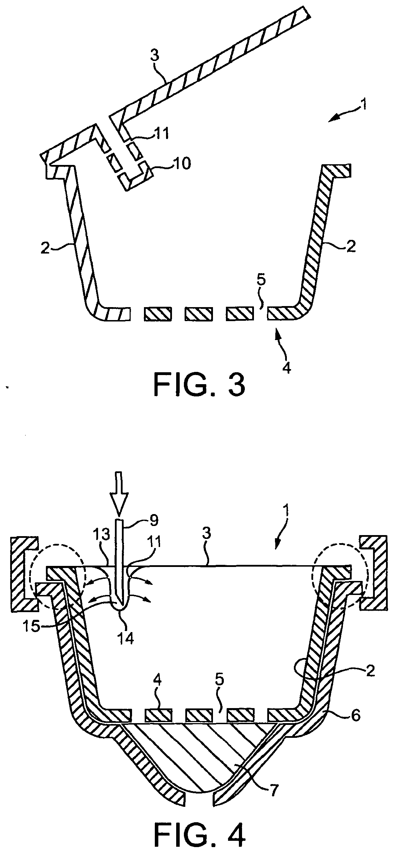

Turning now to **figure 3****,** one can see another variant of the capsule described above in reference to figures 1 and 2. In this case, the top wall 3 is pivotally attached to the capsule side walls 2 via a pivot hinge 12. More precisely, the hinge is disposed between the top wall 3 and the circumferential edge 8. In this case, the capsule bottom wall 4, side walls 2, the hinge 12, and the top wall 3 are manufactured as a single piece by injection molding of a thermoplastic material.

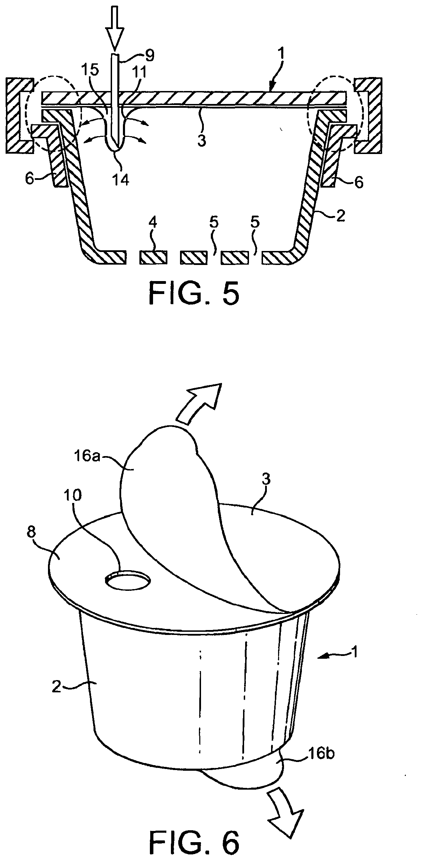

**Figure 4** shows a capsule, wherein the top wall 3 of the capsule 1 is a flexible film. The said film 3 is a multilayer film comprising on the outside a continuous, pierceable layer 13, and the inner layer 14 comprises a deformable layer that is pierced with a plurality of showering holes 11. When the needle 9 presses onto the capsule top wall 3, the first layer 13 of the top wall 3 is pierced by the needle which passes through and contacts the bottom deformable layer 14. Both layers 13, 14 of the top wall delaminate as illustrated in **figures 4** **or** **5****,** such that the deformable layer 14 accommodates the needle shape and size without being pierced through. When fluid is injected through the needle, the fluid occupies the space 15 comprised between the two layers 13, 14, and flows through the showering holes 11 of the deformable layer 14.

At that time, the fluid shower jets that are created inside the capsule have a reduced speed compared to the speed of the fluid jet initially coming out of the needle.

**Figure 5** shows a variant of the capsule described above in reference to figure 4. In this case, the capsule 1 is supported by a ring-shaped capsule holder 6. As described above, the interface between the needle plate, the capsule holder 6, and the capsule edge 8 is leaktight so that fluid injected through the needle 9 can flow only through said capsule.

In this case, it can be seen that no insert supports the bottom wall 4 of the capsule 1, nor does an insert support the side walls 2. Along with the explanations given above, the capsule shown in **figure 5** is therefore meant for a low pressure preparation of a food product.

As illustrated in **figure 6****,** the capsule according to the present invention can be manufactured so that the top wall 3, including the showering means 10, is covered with a peelable film layer 16a sealed onto the circumferential edges 8 of the capsule body. Similarly, the bottom wall 4 can be covered by a peelable film layer 16b, which is sealed onto the circumferential edge of the said bottom wall. Such peelable layers 16a, 16b, avoid the need for a secondary packaging enveloping the whole capsule to keep the product freshness during storage, when the top and/or bottom walls of the capsule are pre-pierced with inlet, respectively outlet openings.

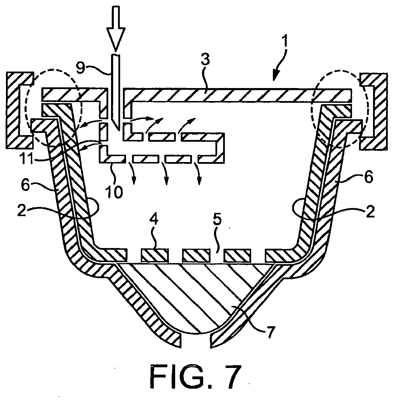

**Figure 7** illustrates an embodiment of the invention, which is a variant of the capsule described above with reference to figure 1. In the present case, the showering protrusion 10 protrudes downwards into the capsule chamber, but its shape is angled so that its distal end extends horizontally across the internal cross-section of the capsule chamber. As can be seen from figure 7, the fluid flow output - marked in the drawing with a series of small arrows - is therefore more even throughout the capsule chamber. Different shapes for the distal end of the showering means protrusion 10 could be envisaged, following the principle of an even distribution of the fluid flow path.

In all embodiments and variants described herein above, the interface between the portion of the needle plate that carries the needle 9, and the top wall 3 of the capsule 1, is leaktight. In this case, the portion of the needle plate surrounding the needle has preferably a shape as described for instance in prior European patent EP 1967099 B1, so as to create a leak-tightness at least locally around the injection point between the needle 9 and the top wall 3.

Alternatively to the leak-tightness achieved locally around the injection point, the sealing can be realised at the interface between the capsule holder upper edge, the capsule circumferential edge 8, and the surroundings of the needle plate, as illustrated by dotted-line circles in figure 5.

In order to guarantee the freshness of the ingredient contained therein, the capsule must be closed during storage, and open only at the time it is used with machine. In a first possibility the capsule itself is open at the top and/or the bottom sides, as represented in **figures 1 to 5****.** The tightness during storage is guaranteed by a secondary packaging such as a flowpack - not shown in the drawing.

Alternatively, the capsule itself is preferably closed, so as to avoid the need for a secondary packaging while guaranteeing the freshness of the product contained inside during long storage periods. In this case, as illustrated in **figure 6****,** the capsule open walls, can be covered with peelable film membrane, which are preferably made of a material barrier to gas and light. The peelable film membranes can be heat sealed, ultrasonically sealed, glued or attached by any other means which provide a tight sealing while allowing a consumer to easily peel them off. In an alternative embodiment - not shown in the drawing -, the peelable film membranes are replaced by rigid or flexible lids which are clipped to the capsule walls.

The capsule according to the present invention is suitable for high or low pressure extraction of the ingredient(s) contained therein. In case the contents is coffee, the capsule of the invention can be used for high pressure extraction of roast and ground powdered coffee beans, or for low pressure dissolution of soluble powdered coffee. The conception of the capsule, independent from the essential features disclosed and claimed in the present patent specification, can be adapted according to the necessary features for high or low pressure use, which the skilled person will be able to choose appropriately. If needed, an insert as described above can be used to guarantee that a high pressure inside the capsule will not damage it.

It should be understood that various changes and modifications to the presently preferred embodiments described herein will be apparent to those skilled in the art. Such changes and modifications can be made without departing from the scope of the present invention and without diminishing its attendant advantages. It is therefore intended that such changes and modifications be covered by the appended claims.

## Claims

1. A capsule (1) for use with a food preparation machine, wherein:
- said machine comprises a fluid injection needle (9) supported by an injection plate, for injection of a single jet of fluid inside the capsule, said needle (9) being adapted in shape and size to protrude inside the capsule (1) when said capsule is inserted into the machine in order for operation, and
- said capsule comprises a body with side (2), bottom (4) and top (3) walls defining a chamber wherein a food ingredient is contained which is to be extracted and/or dissolved by the fluid injected in said chamber through said top wall (3), said capsule further comprising a top circumferential edge (8) such that when said capsule (1) is placed in the machine in order for operation, the interface between the needle plate, and the capsule edge (8) is leaktight so that fluid can flow from the injection means only through said capsule, and
- the capsule top wall (3) is sealed onto said circumferential edge (8),
**characterized in that** the capsule top wall (3) comprises built-in showering means (10) comprising at least one opening (11) so as to accommodate the needle (9) without piercing of said showering means (10), and transform the single jet of fluid from said needle into at least one jet directed towards the inside of the capsule chamber, wherein the showering means (10) comprises a plurality of showering holes (11) having each a diameter which is inferior to the output diameter of the needle (9), and
wherein the showering means (10) comprise a protrusion (10) that protrudes downwards into the capsule chamber and which protrusion (10) comprises an angled shape so that a distal end thereof extends horizontally across the internal cross-section of the capsule chamber.

2. A capsule (1) according to claim 1, wherein the top wall (3) is rigid.

3. A capsule (1) according to claim 2, wherein the top wall (3) is pivotally attached to the capsule side walls (2) via a pivot hinge (12).

4. A capsule (1) according to claim 1, wherein the top wall (3) is a flexible film sealed onto the circumferential edges (8) of the capsule body, said top wall (3) comprising at least one layer (14) of a pre-pierced film that is flexible enough to deform without piercing when in contact with the injection needle.

## Patentansprüche

1. Kapsel (1) zum Gebrauch mit einer Lebensmittelzubereitungsmaschine, wobei:
- die Maschine eine von einer Injektionsplatte getragene Fluidinjektionsnadel (9) zur Injektion eines einzelnen Strahls von Fluid in die Kapsel umfasst, wobei die Nadel (9) in Form und Größe so angepasst ist, dass sie in die Kapsel (1) hinein ragt, wenn die Kapsel zum Betrieb in die Maschine eingeführt wird, und
- die Kapsel einen Körper mit seitlicher (), unterer (4) und oberer (3) Wand umfasst, die eine Kammer definieren, worin eine Lebensmittelzutat enthalten ist, die von dem Fluid, das durch die obere Wand (3) in die Kammer eingespritzt wird, extrahiert und/oder gelöst werden soll, wobei die Kapsel ferner einen oberen Umfangsrand (8) umfasst, so dass, wenn die Kapsel (1) zum Betrieb in die Maschine gegeben wird, die Schnittstelle zwischen der Nadelplatte und dem Kapselrand (8) austrittsdicht ist, so dass Fluid aus dem Injektionsmittel nur durch die Kapsel strömen kann, und
- die obere Kapselwand (3) am Umfangsrand (8) abgedichtet ist,
**dadurch gekennzeichnet, dass** die obere Kapselwand (3) ein eingebautes Beregnungsmittel (10) umfasst, das mindestens eine Öffnung (11) umfasst, um die Nadel (9) aufzunehmen, ohne das Beregnungsmittel (10) zu durchstechen, und den einzelnen Strahl von Fluid aus der Nadel in mindestens einen Strahl umzuwandeln, der zum Inneren der Kapselkammer gerichtet ist, wobei das Beregnungsmittel (10) mehrere Beregnungslöcher (11) mit je einem Durchmesser unterhalb des Ausgabedurchmessers der Nadel (9) umfasst, und
wobei das Beregnungsmittel (10) einen Vorsprung (10) umfasst, der nach unten in die Kapselkammer ragt, und wobei der Vorsprung (10) eine abgewinkelte Form umfasst, so dass ein distales Ende davon horizontal über den Innenquerschnitt der Kapselkammer verläuft.

2. Kapsel (1) nach Anspruch 1, wobei die obere Wand (3) steif ist.

3. Kapsel (1) nach Anspruch 2, wobei die obere Wand (3) mittels eines Drehgelenks (12) schwenkbar an den Kapselseitenwänden (2) befestigt ist.

4. Kapsel (1) nach Anspruch 1, wobei die obere Wand (3) eine flexible Folie ist, die mit den Umfangsrändern (8) des Kapselkörpers verschweißt ist, wobei die obere Wand (3) mindestens eine Schicht (14) einer vorab durchstochenen Folie umfasst, die flexibel genug ist, um sich ohne Durchstechen zu verformen, wenn sie in Kontakt mit der Injektionsnadel ist.

## Revendications

1. Capsule (1) destinée à être utilisée avec une machine de préparation d'aliments, dans laquelle :
- ladite machine comprend une aiguille d'injection de fluide (9) supportée par une plaque d'injection, pour l'injection d'un jet unique de fluide à l'intérieur de la capsule, ladite aiguille (9) étant conçue en forme et en taille pour faire saillie à l'intérieur de la capsule (1) lorsque ladite capsule est insérée dans la machine en ordre pour le fonctionnement, et
- ladite capsule comprend un corps avec des parois latérales (2), inférieure (4) et supérieure (3), définissant une chambre dans laquelle est contenu un ingrédient alimentaire qui doit être extrait et/ou dissous par le fluide injecté dans ladite chambre à travers ladite paroi supérieure (3), ladite capsule comprenant en outre un bord circonférentiel supérieur (8) de telle sorte que, lorsque ladite capsule (1) est placée dans la machine en ordre pour le fonctionnement, l'interface entre la plaque à aiguille, et le bord de capsule (8) est étanche de sorte que le fluide ne peut s'écouler depuis le moyen d'injection qu'à travers ladite capsule, et
- la paroi supérieure de capsule (3) est scellée sur ledit bord circonférentiel (8), **caractérisée en ce que** la paroi supérieure de capsule (3) comprend un moyen d'aspersion intégré (10) comprenant au moins une ouverture (11) de façon à loger l'aiguille (9) sans perforation dudit moyen d'aspersion (10), et à transformer le jet unique de fluide provenant ladite aiguille en au moins un jet dirigé vers l'intérieur de la chambre de capsule, dans lequel le moyen d'aspersion (10) comprend une pluralité de trous d'aspersion (11) ayant chacun un diamètre qui est inférieur au diamètre de sortie de l'aiguille (9), et
dans laquelle les moyens d'aspersion (10) comprennent une partie saillante (10) qui fait saillie vers le bas dans la chambre de capsule et laquelle partie saillante (10) comprend une forme coudée de sorte qu'une extrémité distale de celle-ci s'étend horizontalement à travers la coupe transversale interne de la chambre de capsule.

2. Capsule (1) selon la revendication 1, dans laquelle la paroi supérieure (3) est rigide.

3. Capsule (1) selon la revendication 2, dans laquelle la paroi supérieure (3) est fixée de façon pivotante aux parois latérales de capsule (2) par l'intermédiaire d'une charnière à pivot (12).

4. Capsule (1) selon la revendication 1, dans laquelle la paroi supérieure (3) est un film flexible scellé sur le bord circonférentiel (8) du corps de capsule, ladite paroi supérieure (3) comprenant au moins une couche (14) d'un film pré-perforé qui est suffisamment flexible pour se déformer sans perforation lorsqu'il est en contact avec l'aiguille d'injection.
